# EUROPEAN PATENT APPLICATION

(11) **EP 4 119 102 A1**
(43) Date of publication of application: **18.01.2023**
(21) Application number: 21186251.1
(22) Date of filing: 16.07.2021
(51) Int. Cl.: A61F 2/44, A61F 2/46

(54) **EXPANDABLE AND ANGULARLY ADJUSTABLE SPINAL FUSION CAGES AND ASSOCIATED INSTRUMENTS**

(71) Applicant: Blue Ocean Spine GmbH, 78532 Tuttlingen (DE)
(72) Inventor: SALVERMOSER, Markus, 78532 Möhringen (DE); RICHTER, Jacob, 78532 Tuttlingen (DE); EISEN, Guntmar, 78532 Tuttlingen (DE)
(74) Representative: Stolmár & Partner Patentanwälte PartG mbB

(57) **Abstract**

The present disclosure provides adjustable spinal devices, instruments for implanting the spinal devices, methods for adjusting the height and lordosis angles of the spinal devices and methods for implanting such devices. An adjustable spinal fusion device includes an upper plate component having an outer surface for placement against an endplate of a vertebral body and a lower plate component having an outer surface for placement against an endplate of a vertebral body. The device further includes a first translation member configured to move longitudinally relative to the upper and lower plates to adjust a distance between the upper and lower plates and a second translation member configured to move longitudinally relative to the upper and lower plates to adjust an angle between the upper and lower plates.

## Description

### FIELD

The present disclosure relates to implantable devices for promoting fusion of adjacent bony structures and, more particularly, to implantable spinal fusion cages that can adjust in height and angle to address lordosis within an intervertebral space.

### BACKGROUND

Spinal fusion cages can be used to treat a variety of spinal disorders, including degenerative disc disease. The cages provide a space for inserting a bone graft material between adjacent vertebrae, and to promote fusion between the bones at this spine segment.

A problem common to traditional fusion cages is their lack of ability to maintain or restore the normal anatomy of the fused spine segment. In order to insert the cage between the adjacent vertebra, at least a portion, if not all, of the intervertebral disc is removed to make room for the cage. The removal of the entire disc or disc portion disrupts the normal lordotic or kyphotic curvature of the spine. Traditional fusion cages do not attempt to correct this curvature, and over time as the vertebrae settle around the implanted cages, kyphotic deformity results.

It is therefore desirable to provide spinal fusion cages that have the ability to maintain and restore the normal anatomy of the fused spine segment.

### SUMMARY

The present disclosure provides adjustable spinal devices and instruments for implanting the spinal devices. The present disclosure further provides methods for adjusting the height and lordosis angles of the spinal devices and methods for implanting such devices.

In one embodiment, an adjustable spinal fusion device includes an upper plate component having an outer surface for placement against an endplate of a vertebral body and a lower plate component having an outer surface for placement against an endplate of a vertebral body. The device further includes a first translation member configured to move longitudinally relative to the upper and lower plates to adjust a distance between the upper and lower plates (i.e., the height of the implant); and a second translation member configured to move longitudinally relative to the upper and lower plates to adjust an angle between the upper and lower plates (i.e., the angle of lordosis of the implant).

It is to be understood that both the foregoing general description and the following detailed description are exemplary and explanatory only and are not restrictive of the disclosure. Additional features of the disclosure will be set forth in part in the description which follows or may be learned by practice of the disclosure.

### BRIEF DESCRIPTION OF THE DRAWINGS

The accompanying drawings, which are incorporated in and constitute a part of this specification, illustrate several embodiments of the disclosure and together with the description, serve to explain the principles of the disclosure.

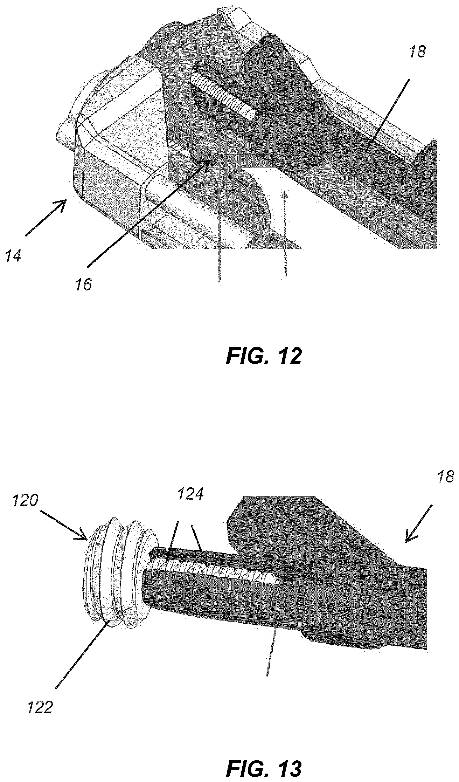

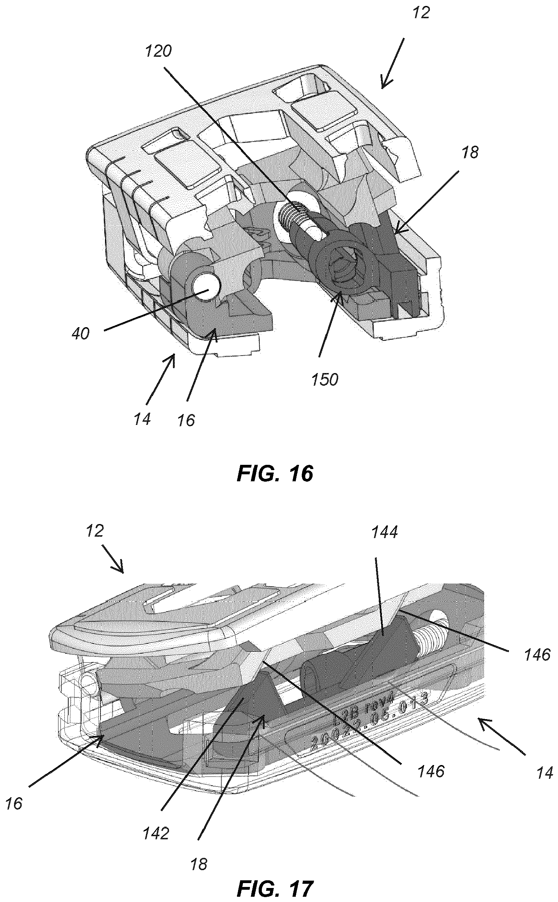

FIGS. 1-18 illustrate various views of an implantable spinal device according to the present disclosure;
FIGS. 19A-19D illustrate adjustment of the height and the angle of the implantable spinal device according to the present disclosure; and
FIGS. 20-26 illustrate various views of an instrument for inserting the implantable spinal device of the present disclosure between adjacent vertebral bodies in a patient.

### DESCRIPTION OF THE EMBODIMENTS

This description and the accompanying drawings illustrate exemplary embodiments and should not be taken as limiting, with the claims defining the scope of the present disclosure, including equivalents. Various mechanical, compositional, structural, and operational changes may be made without departing from the scope of this description and the claims, including equivalents. In some instances, well-known structures and techniques have not been shown or described in detail so as not to obscure the disclosure. Like numbers in two or more figures represent the same or similar elements. Furthermore, elements and their associated aspects that are described in detail with reference to one embodiment may, whenever practical, be included in other embodiments in which they are not specifically shown or described. For example, if an element is described in detail with reference to one embodiment and is not described with reference to a second embodiment, the element may nevertheless be claimed as included in the second embodiment. Moreover, the depictions herein are for illustrative purposes only and do not necessarily reflect the actual shape, size, or dimensions of the system or illustrated components.

It is noted that, as used in this specification and the appended claims, the singular forms "a," "an," and "the," and any singular use of any word, include plural referents unless expressly and unequivocally limited to one referent. As used herein, the term "include" and its grammatical variants are intended to be non-limiting, such that recitation of items in a list is not to the exclusion of other like items that can be substituted or added to the listed items.

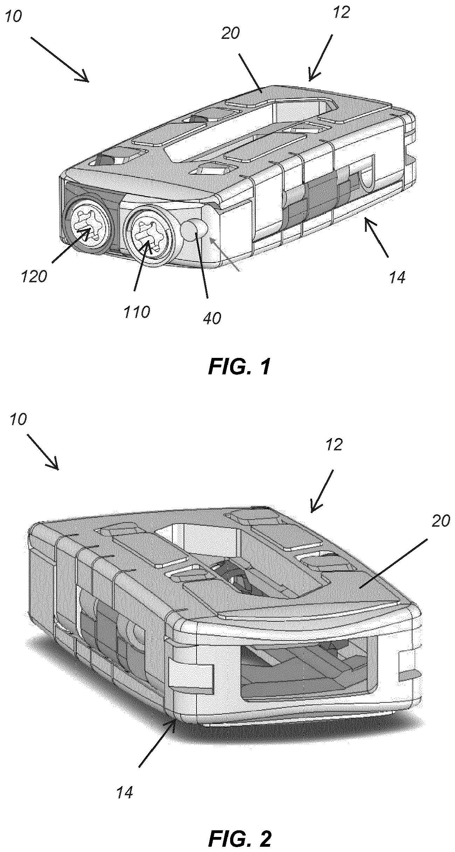

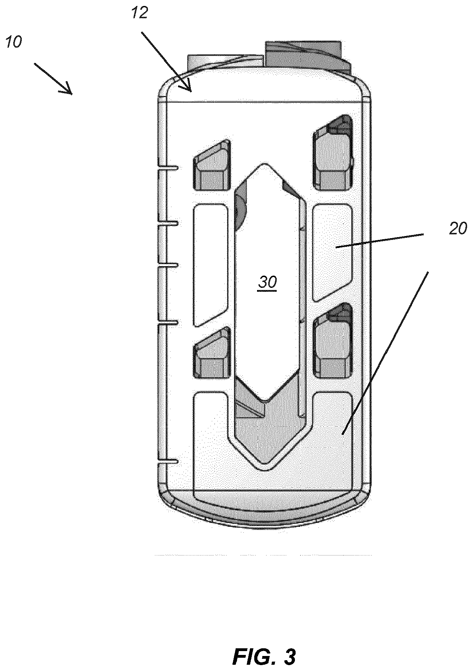

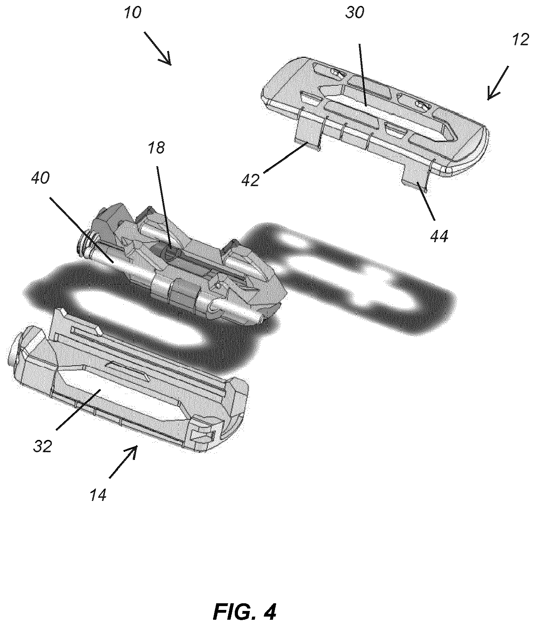

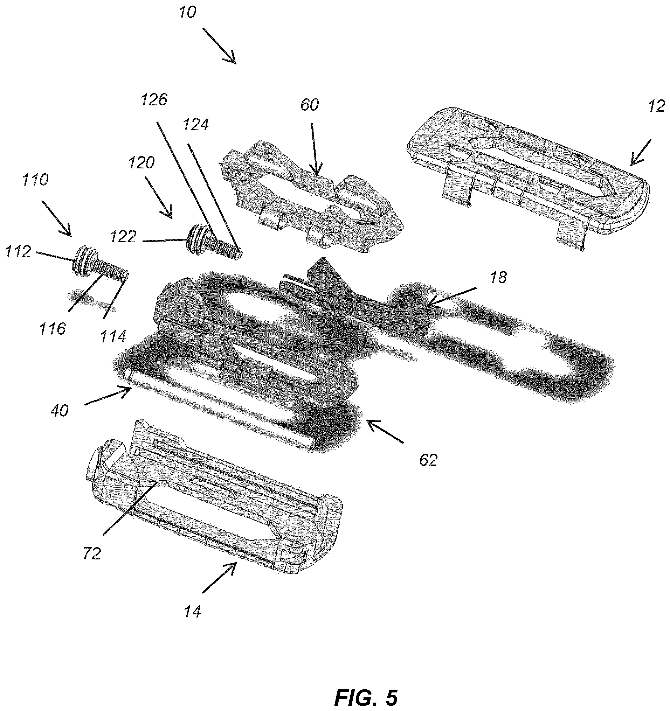

As shown in FIGS. 1-5, a spinal implant 10 according to the present disclosure is configured for placement between two vertebral bodies, preferably from a direct lateral approach through the psoas muscle (lateral lumbar interbody fusion or LLIF). As shown in FIGS. 1-5, the implant includes upper and lower endplates 12, 14, an angle translation member 16 and a height translation member 18 The height and angle translation members 16, 18 are configured to be translated in the longitudinal direction relative to the endplates 12, 14 by first and second shafts 202, 204 of an insertion instrument 200 (discussed below). Movement of the translation members 16, 18 in the longitudinal direction changes the height and angle of the endplates 12, 14 as discussed in more detail below.

The upper and lower endplates 12, 14 each include an outer surface 20, 22, respectively, for contacting the surface of a vertebral body. The outer surfaces 20, 22 are preferably roughened with a surface treatment that facilitates attachment to the vertebral body. The surface treatment preferably creates a diamond structure (e.g., diamond 20-1.5), although other patterns may be used. The upper and lower endplates 12, 14 also include central openings 30, 32 that extend through the entire endplates 12, 14 and, in one embodiment, are substantially aligned with each other. Similarly, the height translation member 18, includes a central opening or bore 34 that, in one embodiment, may be substantially aligned with the endplate openings 30, 32. These openings create space for the addition of bone graft or other substances into the implant, as well as to allow for bony ingrowth through the implant 10.

The upper and lower endplates 12, 14 are connected to each other via a hinge located along one side of the endplates. The hinge comprises a rod 40 in the lower endplate 14 that extends in the longitudinal direction along one side of the upper endplate 12 through first and second knuckles 42, 44 in the upper endplate 12. The knuckles 42, 44 are U-shaped substantially hollow components extending downward from one side of the upper endplate 12. The knuckles 42, 44 allow rotation of the upper endplate 12 relative to the lower endplate 14 around the longitudinal axis defined by the rod 40. In addition, the hollow interior of the knuckles 42, 44 has a larger height than the outer diameter of the rod 40 to allow the upper endplate 12 to move vertically relative to the lower endplate 14, as discussed below.

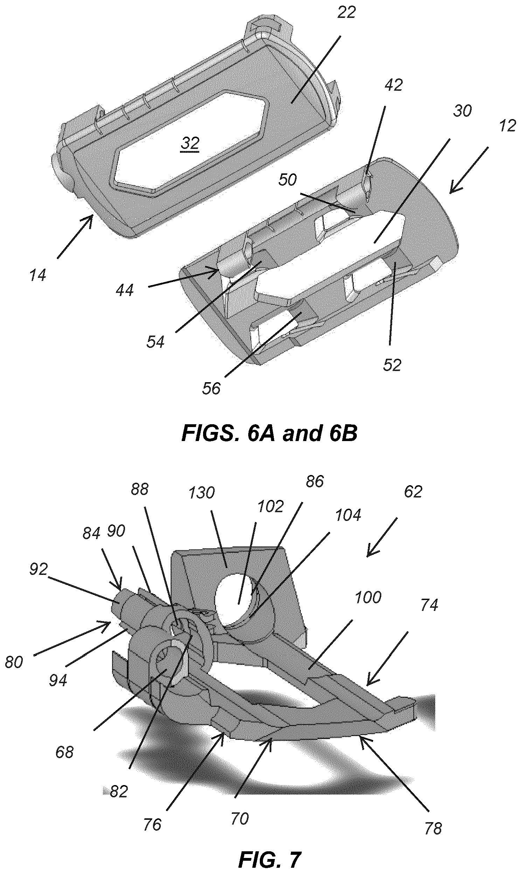

As shown in FIGS. 6A and 6B, the upper endplate includes: (1) first and second distal sloped surfaces or ramps 50, 52 that are laterally spaced from each other near either side of the upper endplate 12 and extend towards the lower endplate 14 in the proximal direction; and (2) third and fourth proximal sloped surfaces or ramps 54, 56 that are laterally spaced from each other near either side of the upper endplate 12 and also extend towards the lower endplate 14 in the proximal direction. These ramps interact with wedges on the height translation member 16 to provide height adjustment of the implant.

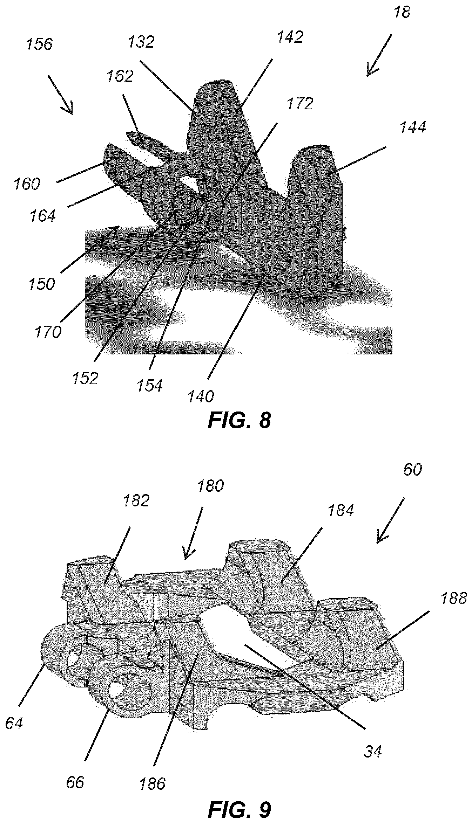

The height translation member 16 includes an upper portion 60 and a lower portion 62 that are separate components from each other. As shown in FIG. 9, the upper portion 60 includes first and second hinge cylinders 64, 66 on one of the sides of the upper portion 60 spaced longitudinally from each other. The hinge cylinders 64, 66 each have a hollow lumen configured to receive the rod 40 of the hinge, which extends through the lumens of the first and second cylinders 64, 66. These cylinders 64, 66 couple the upper portion 60 of the height translation member 16 to the lower endplate 14 and allow this portion to slide longitudinally relative to the endplates along the rod. The lower portion 62 also includes a hinge cylinder 68 on one side of the lower portion 62 that is positioned between the first and second cylinders 64, 66 of the upper portion 60. This cylinder 68 has a hollow lumen that receives the rod 40 of the hinge, thereby coupling the lower portion 60 to the lower endplate 14 and allowing it to slide longitudinally along the rod 40 relative to the endplates.

The upper portion 60 of the height translation member 16 comprises a main body 180 with: (1) first and second distal wedges 182, 184 for engaging with distal ramps 50, 52 on the upper endplate 12 and (2) first and second proximal wedges 186, 188 for engaging with proximal ramps 54, 56 of the upper endplate. Longitudinal movement of translation member 16 causes the upper endplate to move towards and away from the lower endplate.

As shown in FIG. 7, the lower portion 62 of the height translation member 16 comprises a frame 70 generally positioned on, or near, an upper surface 72 of the lower endplate 14. The frame 70 comprises first and second lateral elements 74, 76 that extend longitudinally on either side of the central opening 34 of lower portion 62. The lateral elements 74, 76 are coupled to each other by a proximal base portion 78. The first lateral element 76 comprises a bore 80 with a proximal opening 82 for receiving a first shaft actuator 202 on the insertion instrument 200 and a distal opening 84 for receiving a first ratchet shaft 110 having a threaded head or attachment end.

The proximal portion of the bore 80 includes two recesses 86, 88 formed within the bore 80 and extending laterally outward from the central axis of the bore 80. These recesses 86, 88 are designed to accommodate two projections 204, 206 on the distal end of the first instrument actuator shaft 202. These projections 204, 206 can be rotated into the recesses 86, 88 to lock the instrument shaft 202 to the lower portion 62 of the height translation member 16. Once this occurs, longitudinal translation of the instrument shaft 202 causes longitudinal translation of the lower portion 62 of the height translation member 16.

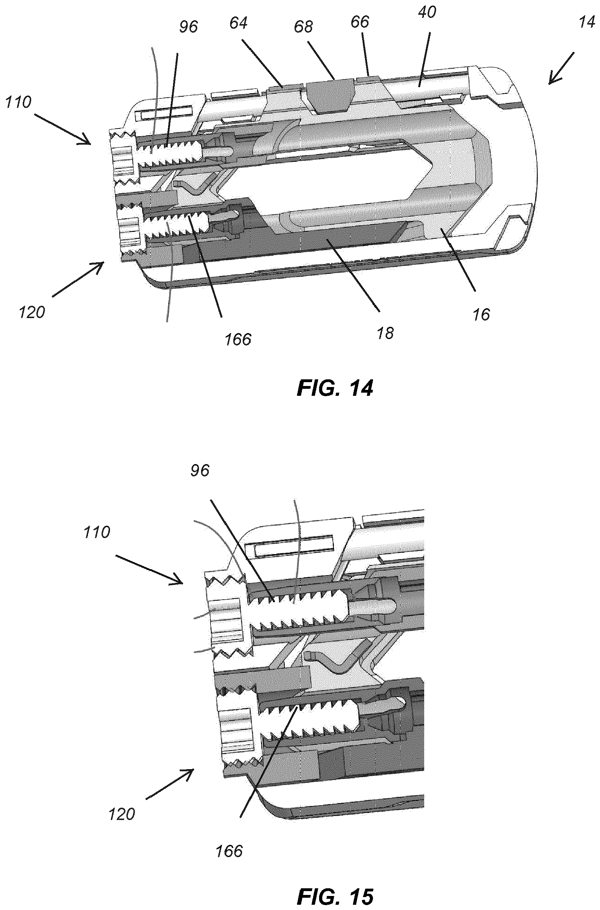

The distal portion of the bore 80 is split into three separate elongate members or fingers 90, 92, 94 that are coupled together at a proximal end and split apart from each other in the distal direction. The three fingers 90, 92, 94 each comprise internal ratchets 96 (see FIG. 14) that mate with a series of external teeth 116 on the first ratchet shaft 110 such that proximal movement of the lower portion 62 of the height translation member 16 causes the internal ratchets 96 to pass over each of the teeth 116 on the ratchet shaft 110 to create discrete "steps" in the movement of the height translation member 16 relative to the endplates 12, 14. These steps correspond to height adjustments or increments of the endplates.

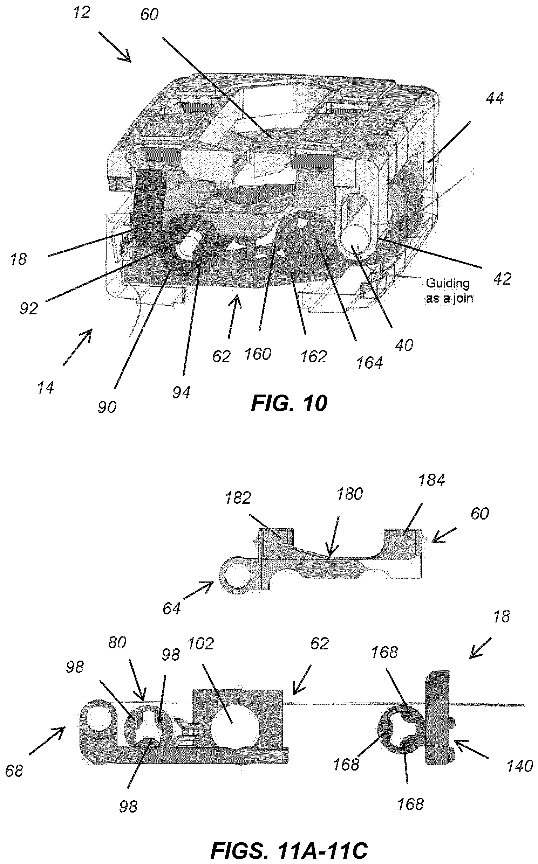

The three fingers 90, 92, 94 generally press inward against the threads 116 of the ratchet shaft 110 to prevent reverse movement of the height translation member 16 in the distal direction. To provide this reverse movement, each of the fingers 90, 92, 94 include internal projections 98 (see FIG. 11A) on their proximal ends designed to mate with a tapered distal end 208 of the instrument actuator shaft. These internal projections 98 are designed such that distal movement of the actuator shaft 202 causes the tapered distal end 208 to engage the projections 98 and urge the fingers laterally outward. As the fingers 90, 92, 94 are urged laterally outward, the internal ratchets 96 disengage from the threads 116 of the ratchet shaft 110 such that distal movement of the height translation member 16 is possible.

The second lateral element 74 of the lower portion 62 of the height translation member 16 has an upper surface 100 and a distal opening or bore 102 with internal threads 104. The angle translation member 18 is positioned on, or near, the upper surface 100 of this lower portion 62. A second ratchet shaft 120 includes a threaded head 122 and a shaft 124 with a series of external teeth 126 extending therefrom. The threaded head 122 of the second ratchet shaft 120 is received within this bore 102.

The lower portion 62 of the height translation member 16 further comprises a distal wedge surface 130 positioned to contact and engage a distal surface 132 of the angle translation member 18. This distal wedge 130 contacts the angle translation member 18 such that, when the height translation member 16 is moved in a proximal direction, it moves the angle translation member 18 therewith.

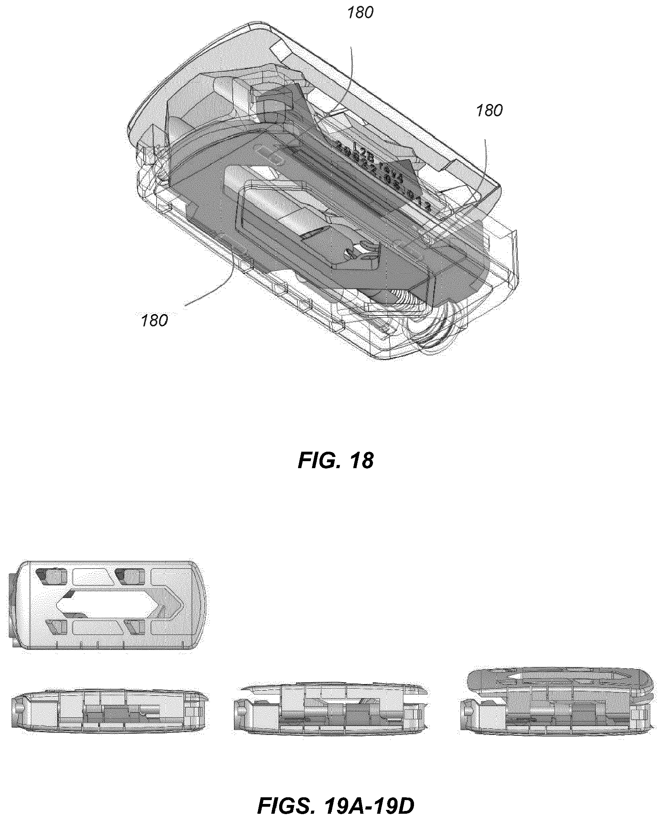

As shown in FIG. 18, the height translation member 16 further comprises runners 180 positioned at the surface of the height translation member adjacent to the horizontal surface of the lower endplate14 and configured to lower the friction therebetween. Alternatively, the runners 180 can also be found within the lower endplate 14.

Referring now to FIG. 8, the angle translation member 18 comprises an elongate body 140 extending in a longitudinal direction and positioned over the upper surface of one of the lateral elements 76 of the lower portion 62 of the height translation member 16. This elongate body 140 includes first and second wedges 142, 144 having a sloped surface that extends downwards in the proximal direction. These wedges 142, 144 are positioned to contact and engage ramps 146 on one side of the upper portion 60 of the height translation member 16. The ramps 146 extend downward from the lower surface of this upper portion 60. Longitudinal movement of the angle translation member 18 causes the wedges 142, 144 to engage the ramps 146 and move one side of the height translation member 16 towards and away from the lower endplate 14. The height translation member 16, in turn, causes one side of the upper endplate 12 to move towards and away from the lower endplate 14. Since the other side of the upper endplate 12 remains fixed in place during this movement, the angle of the upper endplate 12 relative to the lower endplate 14 is thereby adjusted.

The angle translation member 18 further includes a cylindrical component 150 coupled to the elongate body 140 and positioned closer to the longitudinal axis of the implant 10. The cylindrical component 150 includes a central bore 152 with a proximal opening 154 for receiving a second shaft actuator 210 on the insertion instrument 200 and a distal opening 156 for receiving the second threaded ratchet shaft 120.

The distal portion of this bore 152 is split into three separate elongate members or fingers 160, 162, 164 that are coupled together at a proximal end and split apart from each other in the distal direction. The three fingers 160, 162, 164 each comprise internal ratchets 166 (see FIGS. 14 and 15) that mate with the second threaded ratchet shaft 120 such that proximal movement of the angle translation member 18 causes the internal ratchets 166 to pass over each of the threads 126 on the ratchet shaft 120 to create discrete "steps" in the movement of the angle translation member 18 relative to the height translation member 16 and the endplates 12, 14. These steps correlate with angle adjustments of the endplates

The three fingers 160, 162, 165 generally press inward against the second ratchet shaft 120 to prevent reverse movement of the angle translation member 18 in the distal direction. To provide this reverse movement, each of the fingers 160, 162, 164 include internal projections 168 designed to mate with a tapered distal end 216 of the second instrument actuator shaft 210. These internal projections 168 (see FIG. 11C) are designed such that distal movement of the second actuator shaft 210 causes the tapered distal end 216 to engage the projections 168 and urge the fingers 160, 162, 164 laterally outward. As the fingers are urged laterally outward, the internal ratchets 166 disengage from the teeth 126 of the ratchet shaft 120 such that distal movement of the angle translation member 18 is possible.

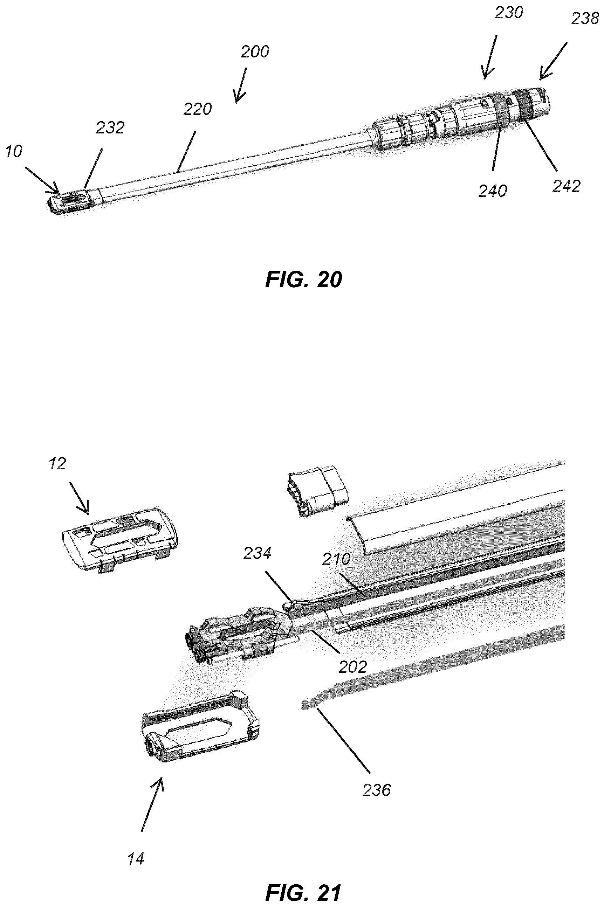

Referring now to FIGS. 20 and 21, the insertion instrument 200 comprises an elongated shaft 220 with a proximal handle 230 and a distal gripping element 232 for removable coupling to the implant. The distal gripping element 232 includes first and second gripping arms 234, 236 for coupling to the lower endplate 14 of the implant (a bayonet style connection). The distal gripping arms 234, 236 are coupled to an actuator 238 on the proximal handle 230 to move the arms 234, 236 in a substantially lateral direction relative to the longitudinal axis of the shaft 220. The arms 234, 236 can be moved together to hold the lower endplate 14 and moved apart to release the endplate.

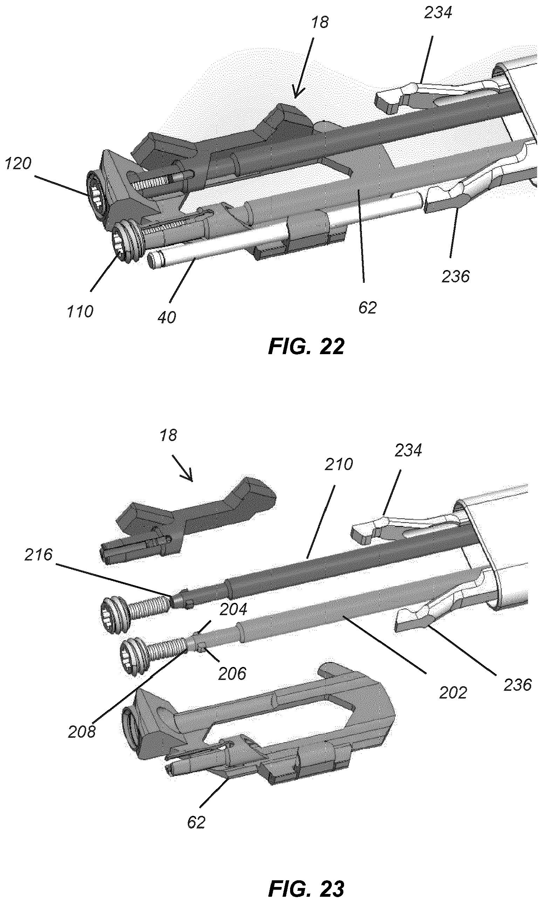

The insertion instrument 200 further includes first and second actuator shafts 202, 210 extending from the handle 230 to the bores 80, 152 within the height and angle translation members 16, 18, respectively. The first and second shafts 202, 210 are positioned on opposite lateral portions of the instrument and are both attached to rotatable knobs 240, 242 on the proximal handle for longitudinally translating the shafts. As shown in FIG. 23, each shaft 202, 210 includes a distal tapered end 208, 216 and at least two projections extending laterally outward from the shaft proximal of the distal tapered end. The two projections are designed to translate through a proximal opening of the bores 80, 152 on the height and angle translation members 16, 18. Rotation of the shafts 202, 210 causes the two projections to slide into the two recesses within these bores, thereby locking the shafts to the height and angle translation members. Rotation of the knobs 240, 242 on the handle then causes the shafts and the translation members to move longitudinally.

The distal tapered end of the instrument shafts are designed to engage the three internal projections within the bores of the angle and height translation members. Proximal movement of the instrument shafts causes the distal tapered end to engage these projections and urge the three elongate distal members of the bores laterally outward to allow reverse or distal movement of the angle and translation members.

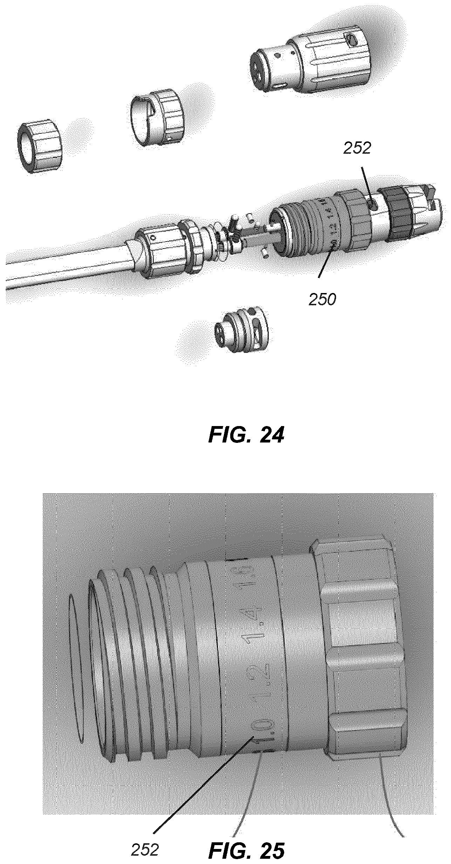

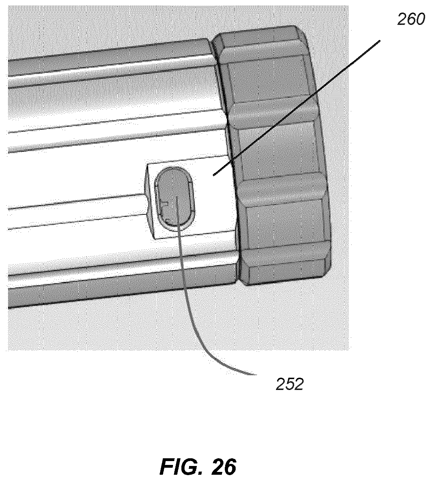

As shown in FIGS. 24-26, the proximal handle 230 comprises position indicator 250, 252 configured to indicate the position of each actuator shaft 202, 210. The position indicators 250, 252 may be formed in a recess 260 of handle 230.

The handle further includes a third rotatable knob 244 coupled to the shaft for rotating the shaft and the endplate therewith relative to the handle. This allows for rotation of the endplate without rotating the handle to facilitate ease of use during implantation.

In use, the implant may be advanced into an intervertebral space in a collapsed configuration (see the resting state shown in FIG. 19A). To increase the height of the implant, the endplates are moved away from each other in a substantially parallel direction. To that end, the first and second instrument shafts are advanced distally through the bores of the angle and height translation members and rotated 90 degrees to lock the shafts to the translation members. The rotatable knob (green) on the handle is rotated to thereby withdraw its associated shaft proximally. This translates both the height and angle translation members in the proximal direction. As the height translation member moves in the proximal direction, its four upper wedges engage with the upper endplate ramps such that the endplates move apart from each other in a substantially parallel direction (see expanded state shown in FIG. 19B).

To adjust the angle of the endplates, the rotatable knob (red) on the handle is rotated, thereby withdrawing its associated shaft proximally. The angle translation member is translated in a proximal direction relative to both the endplates and the height translation member. The wedges on the angle translation member engage the ramps on the one side of the height translation member to move this side upwards away from the lower endplate, thereby adjusting the angle of the upper endplate relative to the lower endplate (see angularly adjusted state shown in FIG. 19C).

The process of height and angle adjustment is reversible. The height and angle may be adjusted independently of each other. For example, the above process can be reversed such that the shaft of the angle translation member is first rotated to adjust angle, and then the shaft of the height translation member is rotated to adjust height.

The entire implant is fabricated through additive manufacturing techniques, such as 3D printing. The implant is formed layer by layer in the longitudinal direction from the proximal end to the distal end. Upon completion of manufacturing, the upper and lower endplates are coupled to each other at certain locations and then separated by a wire EDM process that creates linear cuts to separate the endplates. The hinge rod is machined separately and then inserted through openings in the proximal and distal ends of the lower endplate and through the cylinders in the upper and lower portions of the height translation member.

In an exemplary embodiment, the implants are produced by Selective Laser Melting (SLM). For example, a substrate plate is fastened to an indexing table inside a chamber with a controlled atmosphere of inert gas (e.g., argon or nitrogen). Metal powder is applied flat to the substrate plate as a layer. The metal powder is preferably a titanium alloy, e.g. Ti-6AI-4V to enable biocompatibility. Each 2D slice of the cage is fused by selectively melting the metal powder via a laser. The laser has enough energy to fully melt or rather weld the metal particles to form solid metal. The substrate plate is lowered by the layer thickness (z-direction). New metal powder is applied and the process is repeated layer by layer until the part is complete. The completed part is removed from the substrate plate by cutting or breaking off.

Preferably, all components of the cage are printed nested within each other. Compared to separately 3D printing all components next to each other, a higher utilization rate can be achieved. This means that during 3D printing, a higher proportion which is melted and a lower proportion which stays as metal powder can be achieved. Thus, production time and costs can be reduced significantly.

After 3D printing, areas connecting single components of the cage are cut by electrical discharge machining (EDM) to enable their separate movement. Further, EDM can be used to realize smooth surfaces, e.g., to enable low-friction sliding of two components against each other. With EDM, the cage can also be removed from the substrate plate.

To lower production costs, several cages can be printed onto one substrate plate. In this case, before removing the cages, EDM can be used to simultaneously cut all cages placed on the substrate plate.

The implant may comprise one or more exhaust openings in the upper and lower endplates to allow for extraction of the metal powder remaining in the cage after 3D printing. Preferably, the exhaust opening is positioned on a lateral surface of the moving plate. It is also possible to position the exhaust opening on a horizontal surface of the cage, preferably on the base plate or on the moving plate. Preferably, the cage comprises multiple exhaust openings. Thus, more areas inside the cage are reachable and the metal powder can be extracted more efficiently. It is also possible to configure an external sliding means, preferably a conical groove, in such a way that it can be additionally used as an exhaust opening. Therefore, the conical groove is deepened until a passage to the outside has been made.

Other embodiments will be apparent to those skilled in the art from consideration of the specification and practice of the embodiment disclosed herein. It is intended that the specification and examples be considered as exemplary only, with a true scope and spirit of the embodiment being indicated by the following claims.

## Claims

1. An adjustable spinal fusion device, comprising:
an upper plate component having an outer surface for placement against an endplate of a vertebral body;
a lower plate component having an outer surface for placement against an endplate of a vertebral body;
a first translation member configured to move longitudinally relative to the upper and lower plates to adjust a distance between the upper and lower plates; and
a second translation member configured to move longitudinally relative to the upper and lower plates to adjust an angle between the upper and lower plates.

2. The adjustable spinal fusion device of claim 1, further comprising an internal locking feature to prevent the upper plate from moving relative to the lower plate.

3. The adjustable spinal fusion device of claim 2, wherein the internal locking feature includes teeth for ratcheting.

4. The adjustable spinal fusion device of any preceding claim, wherein the first translation member comprises a mating feature for coupling to a shaft of an insertion instrument such that longitudinal movement of the shaft translates the first translation member in the longitudinal direction.

5. The adjustable spinal fusion device of any preceding claim, wherein the second translation member comprises a mating feature for coupling to a shaft of an insertion instrument such that longitudinal movement of the shaft translates the second translation member in the longitudinal direction.

6. The adjustable spinal fusion device of any preceding claim, wherein the first translation member includes a first movable wedge.

7. The adjustable spinal fusion device of claim 6, wherein the upper endplate comprises a ramp for cooperating with the first movable wedge.

8. The adjustable spinal fusion device of any preceding claim, wherein the second translation member includes a second movable wedge.

9. The adjustable spinal fusion device of claim 8, wherein the first translation member comprises a ramp for cooperating with the second movable wedge.

10. The adjustable spinal fusion device of any preceding claim, further being configured for use in the lumbar spine.
